(19) Europäisches Patentamt
European Patent Office
Office européen des brevets

(11) Veröffentlichungsnummer: **0 033 919**
**B1**

(12) # EUROPÄISCHE PATENTSCHRIFT

(45) Veröffentlichungstag der Patentschrift: **25.05.83**

(21) Anmeldenummer: **81100692.3**

(22) Anmeldetag: **30.01.81**

(51) Int. Cl.³: **C 07 F 9/48,** C 07 F 9/38, C 07 F 9/30, C 07 F 9/65, A 61 K 31/66 // C07D277/16

(54) **Substituierte Phosphin- und Phosphonsäuren, Verfahren und Zwischenprodukte zu ihrer Herstellung, diese enthaltende Arzneimittel und deren therapeutische Verwendung.**

(30) Priorität: **01.02.80 GB 8003420**
**03.12.80 GB 8038795**

(43) Veröffentlichungstag der Anmeldung: **19.08.81 Patentblatt 81/33**

(45) Bekanntmachung des Hinweises auf die Patenterteilung: **25.05.83 Patentblatt 83/21**

(84) Benannte Vertragsstaaten: **AT BE CH DE FR GB IT LI LU NL SE**

(56) Entgegenhaltungen:
**DE-A-2 722 162**
**DERWENT SOVIET INVENTIONS ILLUSTRATED, Section Ch: Chemical, Week C40(1980) Pharmaceuticals, pages 4, 5**

(73) Patentinhaber: **F. HOFFMANN-LA ROCHE & CO. Aktiengesellschaft, CH-4002 Basel (CH)**

(72) Erfinder: **Andrews, Kenneth John Maynard, Hollybush Lane 9, Harpenden Herts. (GB)**

(74) Vertreter: **Lederer, Franz, Dr. et al, Patentanwälte Dr. Franz Lederer Reiner F. Meyer Lucile-Grahn-Strasse 22, D-8000 München 80 (DE)**

**EP 0 033 919 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Substituierte Phosphin- und Phosphonsäure, Verfahren und Zwischenprodukte zu ihrer Herstellung, diese enthaltende Arzneimittel und deren therapeutische Verwendung

Die vorliegende Erfindung betrifft substituierte Phosphin- und Phosphonsäuren. Im speziellen betrifft sie substituierte Phosphin- und Phosphonsäuren, ein Verfahren zu deren Herstellung, pharmazeutische Präparate, enthaltend diese Verbindungen, sowie die Verwendung dieser Verbindungen.

Die erfindungsgemäßen, substituierten Phosphin- und Phonsphonsäuren sind Verbindungen der allgemeinen Formel

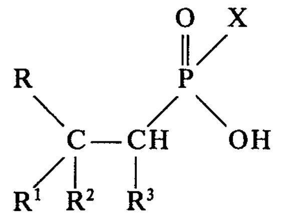

(I)

worin entweder R niederes Alkyl und $R^1$ Wasserstoff oder niederes Alkyl bedeuten, oder R und $R^1$ zusammen mit dem verbindenden Kohlenstoffatom einen 3- bis 6gliedrigen Cycloalkanring bedeuten, und entweder $R^2$ Mercapto und $R^3$ Amino bedeuten, oder $R^2$ und $R^3$ zusammen die Gruppe

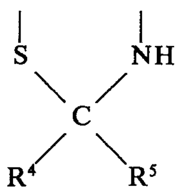

bedeuten, $R^4$ und $R^5$ je niederes Alkyl und X Wasserstoff, Hydroxy, niederes Alkyl oder Aryl bedeuten, und Salze davon.

Die allgemeine Formel I umfaßt demnach Verbindungen der allgemeinen Formel

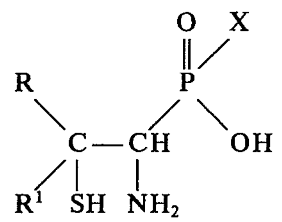

(Ia)

worin R, $R^1$ und X obige Bedeutung besitzen, und Verbindungen der allgemeinen Formel

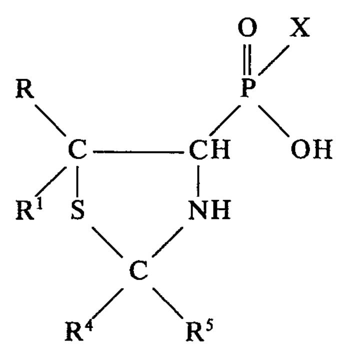

(Ib)

worin R, $R^1$, $R^4$, $R^5$ und X obige Bedeutung besitzen.

Der in dieser Beschreibung verwendete Ausdruck »niederes Alkyl« bezeichnet geradkettige oder verzweigte Alkylgruppen, welche 1 bis 6 Kohlenstoffatome enthalten, wie Methyl, Äthyl, Propyl, Isopropyl, Butyl, Isobutyl, t-Butyl, Pentyl, Hexyl usw. Methyl ist die bevorzugte Bedeutungsmöglichkeit für niederes Alkyl. Phenyl sei als Beispiel für Aryl genannt.

Im Rahmen der vorliegenden Erfindung bevorzugte Verbindungen sind solche, worin R und $R^1$ je niederes Alkyl bedeuten.

Ganz besonders bevorzugte Verbindungen der allgemeinen Formel Ia sind:

DL-(1-Amino-2-mercapto-2-methylpropyl)phosphinsäure,
DL-(1-Amino-2-mercapto-2-methylpropyl)phosphonsäure und
DL-(1-Amino-2-mercapto-2-methylpropyl)methylphosphinsäure.

Weitere bevorzugte Verbindungen der allgemeinen Formel Ia sind:

DL-(1-Amino-2-mercapto-2-methylpropyl)phenylphosphinsäure,
DL-(1-Amino-2-mercapto-2-methylpropyl)butylphosphinsäure,
(−)-(1-Amino-2-mercapto-2-methylpropyl)phosphinsäure und
(+)-(1-Amino-2-mercapto-2-methylpropyl)phosphinsäure.

Ganz besonders bevorzugte Verbindungen der allgemeinen Formel Ib sind:

DL-(2,2,5,5-Tetramethyl-4-thiazolidinyl)phosphinsäure,
DL-(2,2,5,5-Tetramethyl-4-thiazolidinyl)phosphonsäure und
DL-(2,2,5,5-Tetramethyl-4-thiazolidinyl)methylphosphinsäure.

Weitere bevorzugte Verbindungen der allgemeinen Formel Ib sind:

DL-(2,2,5,5-Tetramethyl-4-thiazolidinyl)phenylphosphinsäure und
DL-(2,2,5,5-Tetramethyl-4-thiazolidinyl)butylphosphinsäure.

Die Verbindungen der allgemeinen Formel I und ihre Salze können erfindungsgemäß dadurch hergestellt werden, daß man

a) zur Herstellung eines Säureadditionssalzes einer Verbindung der allgemeinen Formel Ib, ein Säureadditionssalz einer Verbindung der allgemeinen Formel

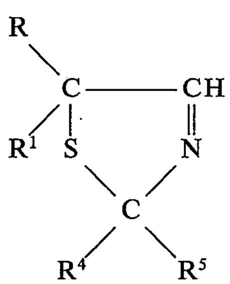

(II)

worin R, $R^1$, $R^4$ und $R^5$ obige Bedeutung besitzen,
mit einer Säure der allgemeinen Formel

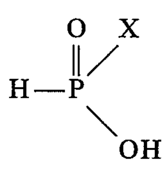

(III)

worin X obige Bedeutung besitzt,
umsetzt, oder

b) zur Herstellung einer Verbindung der allgemeinen Formel Ia oder eines Säureadditionssalzes davon, in einer Verbindung der obigen allgemeinen Formel Ib oder in einer Verbindung der allgemeinen Formel

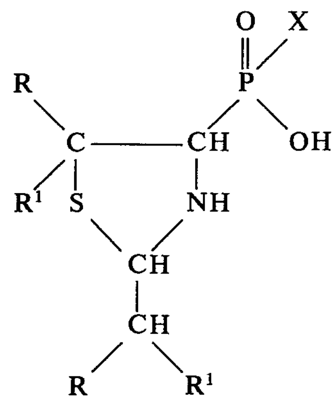

(IV)

worin R, $R^1$ und X obige Bedeutung besitzen,
oder in einem Säureadditionssalz einer Verbindung der allgemeinen Formel Ib oder IV den Thiazolidinring spaltet, oder

c) zur Herstellung einer Verbindung der allgemeinen Formel Ib, eine Verbindung der allgemeinen Formel Ia mit einem Keton der allgemeinen Formel

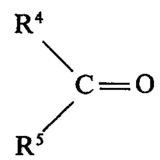

(V)

worin $R^4$ und $R^5$ obige Bedeutung besitzen,
umsetzt, oder

d) erwünschtenfalls ein erhaltenes Diastereoisomerengemisch in die diastereoisomeren Racemate auftrennt, und/oder
e) erwünschtenfalls ein erhaltenes Racemat in die optischen Antipoden spaltet, und/oder
f) erwünschtenfalls eine Verbindung der allgemeinen Formel I in ein Salz überführt, oder ein Salz einer Verbindung der allgemeinen Formel I in eine Verbindung der allgemeinen Formel I überführt.

Gemäß Variante a) des erfindungsgemäßen Verfahrens kann ein Säureadditionssalz einer Verbindung der allgemeinen Formel Ib dadurch erhalten werden, daß man ein Säureadditionssalz einer Verbindung der allgemeinen Formel II mit einer Säure der allgemeinen Formel III umsetzt. In diesem Verfahren verwendet man vorzugsweise ein Säureadditionssalz einer Verbindung der Formel II mit einem Halogenwasserstoff, insbesondere mit Chlorwasserstoff. Die Reaktion kann in An- oder Abwesenheit eines Lösungsmittels durchgeführt werden. In Abwesenheit eines Lösungsmittels können die Reaktionspartner beispielsweise in Form einer Schmelze erhitzt werden. Andererseits kann die Reaktion auch in einem geeigneten Lösungsmittel durchgeführt werden, und zwar beispielsweise in einem Alkohol, wie Äthanol, bei erhöhter Temperatur; z. B. beim Siedepunkt der Mischung oder wenig darunter.

Die Spaltung des Thiazolidinringes gemäß Variante b) des erfindungsgemäßen Verfahrens kann nach allgemein bekannten Methoden durchgeführt werden. Setzt man eine Verbindung der Formel Ib oder ein Säureadditionssalz davon ein, so ist es zweckmäßig, die Spaltung durch Erhitzen einer Lösung oder einer Suspension dieser Verbindung oder eines Salzes davon in Wasser, erwünschtenfalls in Gegenwart einer konzentrierten Halogenwasserstoffsäure, vorzugsweise konzentrierter Salzsäure, durchzuführen. In diesem Fall erhitzt man vorzugsweise auf den Siedepunkt der Reaktionsmischung. Setzt man eine Verbindung der Formel IV oder ein Säureadditionssalz davon ein, so wird die Spaltung zweckmäßigerweise durch Erhitzen dieser Verbindung oder eines Salzes davon in Gegenwart von Wasser und eines mit Wasser nicht mischbaren, inerten organischen Lösungsmittels mit einem Carbonylbindungsmittel durchführt (ein Carbonylbindungsmittel ist eine Verbindung, welche im Stande ist, den während der Reaktion freigesetzten Aldehyd abzufangen). Als Beispiele solcher Cabonylbindungsmittel seien die folgenden genannt: Hydrazin, Phenylhydrazin, 2,4-Dinitrophenylhydrazin, Semicarbazid und Thiosemicarbazid. Ein besonders bevorzugtes Carbonylbindungsmittel ist Hydroxylamin oder ein Salz davon, ganz besonders bevorzugt ist Hydroxylamin-hydrochlorid. Beispiele für mit Wasser nicht mischbare organische Lösungsmittel, welche verwendet werden können, sind die folgenden: Aromatische Kohlenwasserstoffe, wie z. B. Benzol, Toluol und dergleichen, oder vorzugsweise halogenierte Kohlenwasserstoffe, wie z. B. Chloroform, Chlorbenzol und dergleichen. Die Durchführung dieser Spaltungsmethode erfolgt vorteilhafterweise in Gegenwart einer Base, wie eines Alkalimetallhydroxides (z. B. Natriumhydroxid) und beim Siedepunkt der

Reaktionsmischung. Das gewünschte Produkt kann nun in an sich bekannter Weise aus der wäßrigen Phase isoliert werden; das Reaktionsprodukt aus dem Carbonylbindungsmittel und dem Aldehyd verbleibt in der organischen Phase.

Die Reaktion einer Verbindung der Formel Ia mit einem Keton der Formel V nach Variante c) des erfindungsgemäßen Verfahrens zu einer Verbindung der Formel Ib kann in an sich bekannter Weise durchgeführt werden. Als Beispiele für Ketone der Formel V, welche in diesem Verfahren verwendet werden können, seien die folgenden genannt: Aceton, Äthylmethylketon, Diäthylketon und dergleichen. Das bevorzugte Keton ist Aceton.

Die Verbindungen der Formel I besitzen ein asymmetrisches Zentrum und können deshalb in racemischer oder in optisch aktiver Form vorliegen. Verbindungen der Formel I, welche mehr als ein asymmetrisches Zentrum besitzen, können in verschiedenen diastereoisomeren Formen vorliegen. Gegenstand der vorliegenden Erfindung sind alle möglichen Stereoisomeren der Verbindung der Formel I, alle möglichen diastereoisomeren Mischungen und Racemate, sowie die Auftrennung von diastereoisomeren Mischungen und die Spaltung von Racematen, welche nach an sich bekannten Methoden durchgeführt werden können.

Die Verbindungen der Formel I bilden Salze mit Säuren und Basen. Als Beispiele für Säureadditionssalze seien die Hydrochloride, Hydrobromide, Sulfate, Nitrate, Phosphate, Methansulfonate, Äthansulfonate, Toluolsulfonate, Acetate, Oxalate, Succinate, Fumarate, Maleate, Malate und Zitrate genannt. Als Beispiele für Salze mit Basen seien die Natrium-, Kalium-, Lithium-, Calcium-, Ammonium- und substituierte Ammoniumsalze, sowie Salze mit geeigneten heterocyclischen Basen genannt. Bevorzugt werden die pharmazeutisch annehmbaren Salze. Die Überführung einer Verbindung der Formel I in ein Salz kann in herkömmlicher Weise durchgeführt werden. Die Überführung eines Salzes einer Verbindung der Formel I in eine Verbindung der Formel I kann ebenfalls nach herkömmlichen Methoden durchgeführt werden.

Die Verbindungen der Formel II sind bekannt oder können in Analogie zu den bekannten Vertretern dieser Stoffklasse hergestellt werden.

Die Verbindungen der Formel IV und ihre Säureadditionssalze sind neu; sie sind ebenfalls Gegenstand der vorliegenden Erfindung. Sie können dadurch hergestellt werden, daß man eine Verbindung der allgemeinen Formel

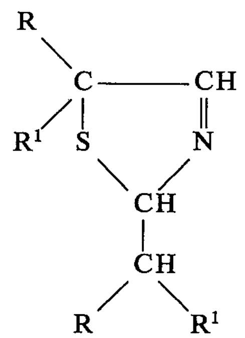

(VI)

worin R und $R^1$ obige Bedeutung besitzen, mit einer Säure der Formel III umsetzt, und zwar in Analogie zu Verfahrensvariante a) und erwünschtenfalls das erhaltene Säureadditionssalz einer Verbindung der Formel IV in eine Verbindung der Formel IV überführt.

Die Verbindungen der Formel I und ihre pharmazeutisch annehmbaren Salze besitzen wertvolle therapeutische Eigenschaften. Sie können insbesondere bei der Behandlung von Entzündungen, degenerativen Gelenkkrankheiten, wie z. B. rheumatoide Arthritis und Osteoarthritis, und der Wilson-Krankheit verwendet werden.

Die Verbindungen der Formel I und ihre pharmazeutisch annehmbaren Salze können als Arzneimittel verwendet werden, und zwar in Form von pharmazeutischen Präparaten, welche diese Verbindungen in Kombination mit einem verträglichen pharmazeutischen Trägermaterial enthalten. Diese Trägermaterialien können für die enterale (z. B. orale) oder parenterale Verabreichung geeignete organische oder anorganische Trägermaterialien sein, wie Wasser, Gelatine, Talk, Magnesiumstearat, Gummi arabicum, Lactose, Stärke, pflanzliche Öle, Polyalkylenglykole, Vaselin und dergleichen. Die pharmazeutischen Präparate können als feste Formen vorliegen, z. B. als Tabletten, Dragées, Suppositorien oder Kapseln, oder in flüssigen Formen, z. B. als Lösungen, Suspensionen oder Emulsionen. Die pharmazeutischen Präparate können herkömmlichen pharmazeutischen Behandlungen, wie Sterilisation, unterzogen werden und/oder können herkömmliche pharmazeutische Hilfsmittel, wie Konservierungsmittel, Stabilisierungsmittel, Emulgiermittel, Netzmittel, Aromatisierungsmittel, Süßmittel, Färbemittel, Salze, um den osmotischen Druck zu variieren, und Puffer enthalten. Die pharmazeutischen Präparate können zusätzlich zu den Verbindungen der allgemeinen Formel I oder ihrer pharmazeutisch annehmbaren Salze auch noch andere therapeutisch wertvolle Substanzen enthalten.

Die Verbindungen der Formel I und ihre pharmazeutisch annehmbaren Salze können Menschen in einer täglichen Dosierung von etwa 250 mg bis etwa 2000 mg verabreicht werden. Die Verabreichung kann in einer einzigen Dosis oder, vorzugsweise in mehreren Dosen erfolgen. Der obige Dosierungsbereich ist nur als Beispiel angegeben und kann durch den behandelnden Arzt nach unten oder nach oben variiert werden, je nach Art und Stärke der zu behandelnden Krankheit und der spezifischen zu verabreichenden Verbindung.

Die nachfolgenden Beispiele dienen der Erläuterung der Erfindung. Alle Temperaturen sind in Celsiusgraden angegeben.

## Beispiel 1

Eine Mischung aus 6,45 g 2,2,5,5-Tetramethyl-$\Delta^3$-thiazolin-hydrochlorid und 2,95 g (1 Äquivalent) phosphorige Säure wird auf 100—110° erhitzt, wobei eine exotherme Reaktion einsetzt, und die Schmelze langsam fest wird. Nach 30 Minuten wird die erhaltene Mischung auf Raumtemperatur abgekühlt und mit Äthanol versetzt. Der pH der erhaltenen Suspension wird mit Propylenoxid auf 5—6 eingestellt. Der Niederschlag wird abfiltriert, mit Äthanol und Diäthyläther gewaschen und getrocknet. Man erhält 6,0 g DL-(2,2,5,5-Tetramethyl-4-thiazolidinyl)phosphonsäure als weißen Festkörper vom Schmelzpunkt 235—237° (Zersetzung).

Das als Ausgangsmaterial verwendete 2,2,5,5-Tetramethyl-$\Delta^3$-thiazolin-hydrochlorid kann wie folgt hergestellt werden:

Eine in einem Eisbad abgekühlte Lösung von 5,2 g 2,2,5,5,-Tetramethyl-$\Delta^3$-thiazolin in trockenem Diäthyläther wird bis zur Vervollständigung des Niederschlages mit trockenem Chlorwasserstoff behandelt. Der Niederschlag wird abfiltriert, mit Diäthyläther gewaschen und im Vakuumexsikkator getrocknet.

## Beispiel 2

Eine Lösung von 36,0 g 2,2,5,5-Tetramethyl-$\Delta^3$-thiazolin-hydrochlorid in Äthanol wird mit 13,0 g (1 Äquivalent) hypophosphorige Säure versetzt. Die Lösung wird während 3 Stunden auf 70° erwärmt und anschließend filtriert. Das Filtrat wird auf Raumtemperatur abgekühlt, mit Propylenoxid auf pH 5 gestellt und während 4 Stunden auf 0° abgekühlt. Der Festkörper wird abfiltriert, mit Äthanol/Diäthyläther gewaschen und getrocknet. Man erhält 34,0 g DL-(2,2,5,5-Tetramethyl-4-thiazolidinyl)phosphinsäure vom Schmelzpunkt 191° (Zersetzung).

## Beispiel 3

7,2 g 2,2,5,5-Tetramethyl-$\Delta^3$-thiazolin-hydrochlorid und 3,25 g (1 Äquivalent) Methylphosphinsäure werden gründlich vermischt. Die Mischung wird anschließend unter Rühren auf 105—110° erhitzt. Es bildet sich eine Schmelze, wobei ein saures Gas entsteht. Die Schmelze wird langsam fest und man erhitzt noch während 10—15 Minuten. Der Rückstand wird mit 3,0 g Propylenoxid in Äthanol behandelt, anschließend läßt man die Lösung noch während 12 Stunden stehen. Der Festkörper wird abfiltriert, mit kaltem Isopropanol/Diäthyläther gewaschen und getrocknet. Nach Umkristallisieren aus Isopropanol erhält man 2,1 g DL-(2,2,5,5-Tetramethyl-4-thiazolidinyl)methylphosphinsäure als weißen Festkörper vom Schmelzpunkt 193—194°.

## Beispiel 4

2,0 g DL-(2,2,5,5-Tetramethyl-4-thiazolidinyl)phosphonsäure (hergestellt, wie in Beispiel 1 beschrieben) werden in siedendem Wasser aufgelöst. Die erhaltene Lösung wird bis zur beginnenden Kristallisation eingeengt. Die Mischung wird anschließend auf 0° abgekühlt. Die weißen Kristalle werden abfiltriert. Man erhält 0,6 g DL-(1-Amino-2-mercapto-2-methylpropyl)phosphonsäure vom Schmelzpuntk 242—244° (Zersetzung).

## Beispiel 5

0,5 g DL-(2,2,5,5-Tetramethyl-4-thiazolidinyl)phosphinsäure (hergestellt, wie in Beispiel 2 beschrieben) werden in siedendem Wasser aufgelöst. Die erhaltene Lösung wird eingeengt und auf 0° abgekühlt. Nach Abfiltrieren des weißen, kristallinen Festkörpers erhält man 0,15 g DL-(1-Amino-2-mercapto-2-methylpropyl)phosphinsäure vom Schmelzpunkt 205—207° (Zersetzung).

Beispiel 6

1,0 g DL-(2,2,5,5-Tetramethyl-4-thiazolidinyl)methylphosphinsäure (hergestellte, wie in Beispiel 3 beschrieben) werden in siedendem Wasser gelöst. Die Lösung wird während 30 Minuten zum Sieden erhitzt und anschließend eingeengt. Die Lösung wird anschließend filtriert und mit Isopropanol versetzt. Nach Abfiltrieren des kristallinen Produkts, Waschen mit Isopropanol/Diäthyläther und Trocknen erhält man 0,7 g DL-(1-Amino-2-mercapto-2-methylpropyl)methylphosphinsäure als weißen Festkörper vom Schmelzpunkt 221°.

Beispiel 7

0,54 g 2,2,5,5-Tetramethyl-$\Delta^3$-thiazolin-hydrochlorid und 0,42 g Phenylphosphinsäure werden zusammen mit 10 ml Äthanol während 2 Stunden auf 70° erhitzt. Die Mischung wird auf Raumtemperatur abgekühlt und filtriert. Der Rückstand wird mit Äthanol und Diäthyläther gewaschen und anschließend getrocknet. Man erhält 0,65 g DL-(2,2,5,5-Tetramethyl-4-thiazolidinyl)phenylphosphinsäure-hydrochlorid vom Schmelzpunkt 215–217° (Zersetzung).

Beispiel 8

7,73 g 2,2,5,5-Tetramethyl-$\Delta^3$-thiazolin-hydrochlorid und 5,27 g Butylphosphinsäure werden zusammen vermischt und langsam auf 100° erwärmt. Nach Einsetzen der Reaktion steigt die Temperatur auf 130°; man erhitzt noch während 0,5 Stunden. Das erhaltene feste Gemisch wird mit Methanol behandelt. Der Festkörper wird abfiltriert, mit Methanol gewaschen und aus Methanol umkristallisiert. Man erhält 5,66 g DL-(2,2,5,5-Tetramethyl-4-thiazolidinyl)butylphosphinsäure-hydrochlorid vom Schmelzpunkt 204° (Zersetzung).

Beispiel 9

0,3 g DL-(2,2,5,5-Tetramethyl-4-thiazolidinyl)phenylphosphinsäure-hydrochlorid (hergestellt, wie in Beispiel 7 beschrieben) wird in siedendem Wasser suspendiert. Man versetzt mit konzentrierter Salzsäure bis eine Lösung entsteht. Die Lösung wird filtriert und unter vermindertem Druck zur Trockne eingedampft. Der feste Rückstand wird aus 3N Salzsäure umkristallisiert und liefert DL-(1-Amino-2-mercapto-2-methylpropyl)phenylphosphinsäure-hydrochlorid vom Schmelzpunkt 190–192° (Zersetzung).

Beispiel 10

7,7 g DL-(2,2,5,5-Tetramethyl-4-thiazolidinyl)butylphosphinsäure (hergestellt, wie in Beispiel 8 beschrieben) werden in 25 ml Wasser, das 5 ml konzentrierte Salzsäure enthält, suspendiert. Die Mischung wird während 1 Stunde unter Rückfluß zum Sieden erhitzt. Nach Eindampfen zur Trockne versetzt man einmal mit Äthanol und dampft ein. Der feste Rückstand wird aus einer Mischung von 20 ml Äthanol und 75 ml Diäthyläther umkristallisiert. Das kristalline Produkt wird abfiltriert, wobei man 4,1 g DL-(1-Amino-2-mercapto-2-methylpropyl)butylphosphinsäurehydrochlorid vom Schmelzpunkt 159–160° (Zersetzung) erhält.

Beispiel 11

2,23 g racemische (2-Isopropyl-5,5-dimethyl-4-thiazolidinyl)phosphinsäure und 0,77 g Hydroxylamin-hydrochlorid werden in 15 ml sauerstofffreiem Wasser, das 0,48 g Natriumhydroxid enthält, aufgelöst. Nach Zugabe von 15 ml Chloroform wird die Mischung während 1 Stunde unter Rückfluß zum Sieden erhitzt, auf Raumtemperatur abgekühlt und mit 3 ml konzentrierter Salzsäure versetzt. Nach Abtrennen der Chloroformphase wird die wäßrige Phase zweimal mit je 15 ml Chloroform ausgeschüttelt. Die wäßrige Phase wird filtriert und zur Trockne eingedampft. Den erhaltenen Rückstand versetzt man zweimal mit Äthanol und dampft jeweils wieder ein. Der Rückstand wird in 15 ml Äthanol suspendiert und mit 10 ml Aceton behandelt. Die Mischung wird während 0,5 Stunden unter Rückfluß zum Sieden erhitzt, auf Raumtemperatur abgekühlt und filtriert. Das Filtrat wird mit 1,5 ml Propylenoxid behandelt und anschließend während 2 Stunden auf 0–5° abgekühlt. Der erhaltene Festkörper wird abfiltriert, mit Isopropanol gewaschen und getrocknet. Man erhält 1,5 g DL-(2,2,5,5-Tetramethyl-4-thiazolidinyl)phosphinsäure vom Schmelzpunkt 190° (Zersetzung).

Die als Ausgangsmaterial verwendete racemische (2-Isopropyl-5,5-dimethyl-4-thiazolidinyl)phos-

phinsäure kann wie folgt hergestellt werden:

Eine Lösung von 31,6 g 2-Isopropyl-5,5-dimethyl-$\Delta^3$-thiazolin in 300 ml trockenem Diäthyläther wird in einem Eisbad abgekühlt und bis nichts mehr ausfällt mit trockenem Chlorwasserstoff behandelt. Der Festkörper wird abfiltriert, mit trockenem Diäthyläther gewaschen und getrocknet. Man erhält 35 g 2-Isopropyl-5,5-dimethyl-$\Delta^3$-thiazolinhydrochlorid.

Eine Lösung von 19,4 g 2-Isopropyl-5,5-dimethyl-$\Delta^3$-thiazolin-hydrochlorid und 16,0 g hypophosphorige Säure in 75 ml Äthanol wird während 1 Stunde auf 70° erwärmt. Nach Abkühlen auf Raumtemperatur und Filtrieren wird die erhaltene Lösung mit 7 ml Propylenoxid behandelt und während 3 Stunden auf 0–5° abgekühlt. Der erhaltene Festkörper wird abfiltriert, mit Äthanol und Diäthyläther gewaschen und getrocknet. Man erhält 18,4 g racemische (2-Isopropyl-5,5-dimethyl-4-thiazolidinyl)phosphinsäure vom Schmelzpunkt 200° (Zersetzung).

Beispiel 12

1,5 g DL-(2,2,5,5-Tetramethyl-4-thiazolidinyl)phosphinsäure werden in 10 ml siedendem Wasser aufgelöst. Die erhaltene Lösung wird während 0,25 Stunden bei Siedetemperatur eingeengt, auf Raumtemperatur abgekühlt und mit 25 ml Isopropanol verdünnt. Die erhaltene Mischung wird über Nacht bei 0° gehalten. Der entstandene Festkörper wird abfiltriert, mit Isopropanol gewaschen und getrocknet. Man erhält 0,85 g DL-(1-Amino-2-mercapto-2-methylpropyl)-phosphinsäure vom Schmelzpunkt 211° (Zersetzung).

Beispiel 13

a) 1,7 g DL-(1-Amino-2-mercapto-2-methylpropyl)phosphinsäure werden in 50 ml sauerstofffreiem Wasser, das 1,7 g Natriumbicarbonat enthält, aufgelöst. Die Lösung wird unter einer Argonatmosphäre bei 0° gehalten. Man versetzt mit 1,7 ml Benzylchloroformiat und rührt die Mischung während 2 Stunden, wobei man die Temperatur während dieser Zeit langsam auf Raumtemperatur steigen läßt. Nach Extrahieren mit 20 ml Diäthyläther wird die wäßrige Phase abgetrennt, auf 0° abgekühlt und mit 2N Salzsäure auf pH 1 angesäuert. Das Öl, das sich langsam abscheidet, verfestigt sich und wird aus Essigester/Petroläther umkristallisiert. Man erhält DL-(1-Benzyloxycarbonylamino-2-mercapto-2-methylpropyl)phosphinsäure vom Schmelzpunkt 114–120°.

b) Eine heiße Lösung von 6,36 g DL-(1-Benzyloxycarbonylamino-2-mercapto-2-methylpropyl)phosphinsäure in 20 ml Äthanol wird mit einer Lösung von 2,52 g (–)-$\alpha$-Methylbenzylamin in 5 ml Äthanol behandelt. Man läßt die Lösung langsam abkühlen und filtriert das erhaltene kristalline Produkt ab. Man erhält 1,60 g des (–)-$\alpha$-Methylbenzylaminsalzes der (+)-(1-Benzyloxycarbonylamino-2-mercapto-2-methylpropyl)phosphinsäure vom Schmelzpunkt 185–189°; $[\alpha]_D^{20} = +22{,}2°$ (c = 1% in Äthanol). Das Produkt wird bis zur konstanten Drehung umkristallisiert: $[\alpha]_D^{20} = +24{,}6°$ (c = 1% in Äthanol), Schmelzpunkt 187–189°.

c) Die nach den Umkristallisationen des (–)-$\alpha$-Methylbenzylaminsalzes in Absatz b) erhaltenen Mutterlaugen werden eingedampft. Der feste Rückstand wird in Wasser suspendiert und mit 2N Salzsäure auf pH 1 angesäuert. Die Mischung wird zweimal mit je 25 ml Diäthyläther extrahiert. Die vereinigten Auszüge werden über Magnesiumsulfat getrocknet, filtriert und eingedampft. Der erhaltene Stoff wird mit (+)-$\alpha$-Methylbenzylamin behandelt, und zwar wie in Absatz b) beschrieben. Man erhält das kristalline (+)-$\alpha$-Methylbenzylaminsalz der (–)-(1-Benzyloxycarbonylamino-2-mercapto-2-methylpropyl)phosphinsäure, das bis zur konstanten Drehung aus Äthanol umkristallisiert wird. Man erhält Produkt vom Schmelzpunkt 187–189° und $[\alpha]_D^{20} = -23{,}9°$ (c = 1% in Äthanol).

d) Das (+)-$\alpha$-Methylbenzylaminsalz der (–)-(1-Benzyloxycarbonylamino-2-mercapto-2-methylpropyl)phosphinsäure [Absatz c)] wird in Wasser suspendiert. Die Suspension wird mit 2N Salzsäure auf pH 1 angesäuert und zweimal mit 25 ml Diäthyläther ausgeschüttelt. Die vereinigten ätherischen Auszüge werden über Magnesiumsulfat getrocknet und eingedampft. Das erhaltene Öl wird in 2 ml Eisessig aufgelöst und mit 5 ml 45prozentigem Bromwasserstoff in Essigsäure behandelt. Man läßt die erhaltene Lösung über Nacht bei Raumtemperatur stehen, versetzt anschließend mit 200 ml Diäthyläther und läßt die trübe Suspension während 2 Stunden bei 0° stehen. Die ätherische Phase wird abdekantiert, und der Rückstand in 15 ml Methanol aufgelöst. Nach Zugabe von 10 ml Aceton wird die Lösung während 1 Stunde unter Rückfluß zum Sieden erhitzt, abgekühlt und filtriert. Das Filtrat wird durch Zugabe von Propylenoxid auf pH 5 eingestellt. Das Acetonaddukt kristallisiert bei 0° aus und wird abfiltriert. Das Acetonaddukt, das einen Schmelzpunkt von 183–185° besitzt, wird in 5 ml siedendem Wasser aufgelöst. Die Lösung wird filtriert und durch Abdampfen von überschüssigem Wasser auf 3 ml eingedampft, mit 15 ml Isopropanol versetzt und langsam auf Raumtemperatur abgekühlt. Anschließend wird die Mischung während 16 Stunden bei 0° gehalten. Der erhaltene kristalline Festkörper wird abfiltriert

und getrocknet, wobei man (−)-(1-Amino-2-mercapto-2-methylpropyl)phosphinsäure vom Schmelzpunkt 211−212° (Zersetzung) und $[\alpha]_D^{25} = -4{,}5°$ (c=1,5% in Wasser) erhält.

e) Das (−)-α-Methylbenzylaminsalz der (+)-(1-Benzyloxycarbonylamino-2-mercapto-2-methylpropyl)phosphinsäure [Absatz b)] wird in Analogie zu den Angaben in Absatz d) behandelt. Man erhält (+)-(1-Amino-2-mercapto-2-methylpropyl)phosphinsäure vom Schmelzpunkt 215° (Zersetzung) und $[\alpha]_D^{25} = +4{,}0°$ (c=1,5% in Wasser).

Die folgenden Beispiele beschreiben pharmazeutische Präparate, enthaltend eine Verbindung der Formel I als Wirkstoff:

Beispiel A

Tabletten können die folgenden Bestandteile enthalten:

| Bestandteile | mg/Tablette |
|---|---|
| Verbindung der Formel I | 100,0 |
| Lactose | 298,0 |
| Maisstärke | 80,0 |
| Magnesiumstearat | 2,0 |
| Gesamtgewicht | 480,0 |

Beispiel B

Tabletten können die folgenden Bestandteile enthalten:

| Bestandteile | mg/Tablette |
|---|---|
| Verbindung der Formel I | 250,0 |
| Lactose | 96,0 |
| Magnesiumstearat | 4,0 |
| Maisstärke | 75,0 |
| Hydroxypropylmethylcellulose | 25,0 |
| Gesamtgewicht | 450,0 |

**Patentansprüche für die Vertragsstaaten BE, CH, DE, FR, GB, IT, LI, LU, NL, SE**

1. Substituierte Phosphin- und Phosphonsäuren der allgemeinen Formel

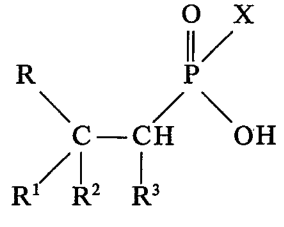

(I)

worin entweder R niederes Alkyl und $R^1$ Wasserstoff oder niederes Alkyl bedeuten, oder R und $R^1$ zusammen mit dem verbindenden Kohlenstoffatom einen 3- bis 6gliedrigen Cycloalkanring bedeuten,

und entweder $R^2$ Mercapto und $R^3$ Amino bedeuten, oder $R^2$ und $R^3$ zusammen die Gruppe

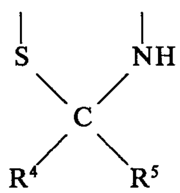

bedeuten, $R^4$ und $R^5$ je niederes Alkyl und X Wasserstoff, Hydroxy, niederes Alkyl oder Aryl bedeuten, und Salze davon in Form von diastereoisomeren Mischungen, Racematen und optisch einheitlichen Stoffen.

2. Verbindungen der allgemeinen Formel

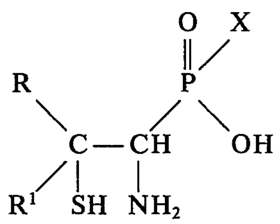

(Ia)

worin R, $R^1$ und X die in Anspruch 1 angegebene Bedeutung besitzen.

3. Verbindungen der allgemeinen Formel

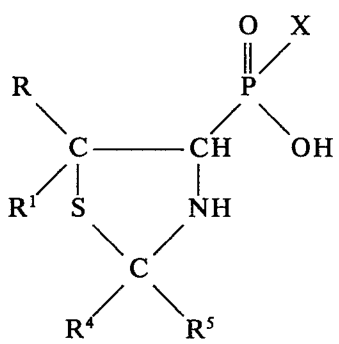

(Ib)

worin R, $R^1$, $R^4$, $R^5$ und X die in Anspruch 1 angegebene Bedeutung besitzen.

4. Verbindungen gemäß einem der Ansprüche 1 bis 3, dadurch gekennzeichnet, daß entweder R niederes Alkyl und $R^1$ Wasserstoff oder niederes Alkyl bedeuten, oder R und $R^1$ zusammen Trimethylen, Tetramethylen oder Pentamethylen bedeuten.

5. Verbindungen gemäß einem der Ansprüche 1 bis 4, dadurch gekennzeichnet, daß R und $R^1$ je niederes Alkyl bedeuten.

6. DL-(1-Amino-2-mercapto-2-methylpropyl)phosphinsäure.

7. DL-(1-Amino-2-mercapto-2-methylpropyl)phosphonsäure.

8. DL-(1-Amino-2-mercapto-2-methylpropyl)methylphosphinsäure.

9. DL-(2,2,5,5-Tetramethyl-4-thiazolidinyl)phosphinsäure.

10. DL-(2,2,5,5-Tetramethyl-4-thiazolidinyl)phosphonsäure.

11. DL-(2,2,5,5-Tetramethyl-4-thiazolidinyl)methylphosphinsäure.

12. DL-(1-Amino-2-mercapto-2-methylpropyl)phenylphosphinsäure, DL-(1-Amino-2-mercapto-2-methylpropyl)butylphosphinsäure, (−)-(1-Amino-2-mercapto-2-methylpropyl)phosphinsäure, (+)-(1-Amino-2-mercapto-2-methylpropyl)phosphinsäure, DL-(2,2,5,5-Tetramethyl-4-thiazolidinyl)phenylphosphinsäure und DL-(2,2,5,5-Tetramethyl-4-thiazolidinyl)butylphosphinsäure.

13. Verbindungen der allgemeinen Formel

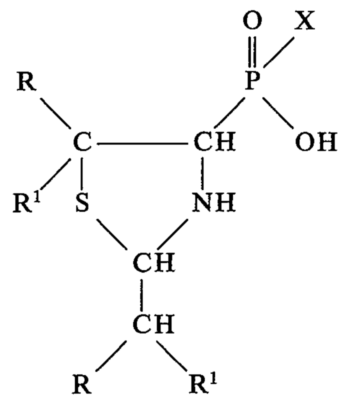

(IV)

worin entweder R niederes Alkyl und $R^1$ Wasserstoff oder niederes Alkyl bedeuten, oder R und $R^1$ zusammen mit dem verbindenden Kohlenstoffatom einen 3- bis 6gliedrigen Cycloalkanring bedeuten, und X Wasserstoff, Hydroxy, niederes Alkyl oder Aryl bedeutet,
und Säureadditionssalze davon.

14. Racemische (2-Isopropyl-5,5-dimethyl-4-thiazolidinyl)phosphinsäure.

15. Verbindungen gemäß einem der Ansprüche 1 bis 12 zur Anwendung als pharmazeutische Wirkstoffe.

16. Verbindungen gemäß einem der Ansprüche 1 bis 12 als pharmazeutische Wirkstoffe zur Behandlung von Entzündungen, degenerativen Gelenkkrankheiten oder der Wilson-Krankheit.

17. Verfahren zur Herstellung von Verbindungen gemäß einem der Ansprüche 1 bis 12, dadurch gekennzeichnet, daß man

a) zur Herstellung eines Säureadditionssalzes einer Verbindung der allgemeinen Formel

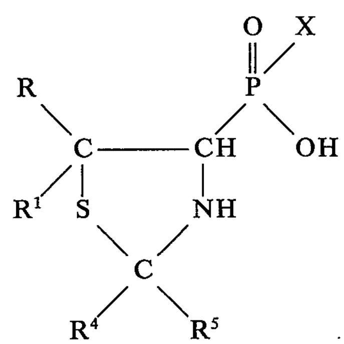

(Ib)

worin R, $R^1$, $R^4$, $R^5$ und X die in Anspruch 1 angegebene Bedeutung besitzen,
ein Säureadditionssalz einer Verbindung der allgemeinen Formel

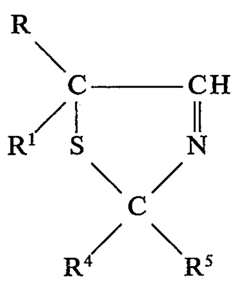

(II)

worin R, $R^1$, $R^4$ und $R^5$ die in Anspruch 1 angegebene Bedeutung besitzen,
mit einer Säure der allgemeinen Formel

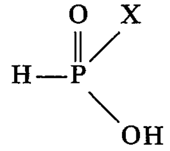

(III)

worin X die in Anspruch 1 angegebene Bedeutung besitzt, umsetzt, oder

b) zur Herstellung einer Verbindung der allgemeinen Formel

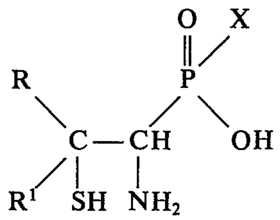

(Ia)

worin R, $R^1$ und X die in Anspruch 1 angegebene Bedeutung besitzen, oder von Säureadditionssalzen davon, in einer Verbindung der Formel Ib oder in einer Verbindung der allgemeinen Formel

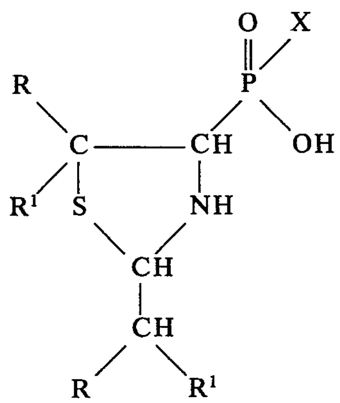

(IV)

worin R, $R^1$ und X die in Anspruch 1 angegebene Bedeutung besitzen, oder in einem Säureadditionssalz einer Verbindung der Formel Ib oder IV den Thiazolidinring spaltet, oder

c) zur Herstellung einer Verbindung der obigen allgemeinen Formel Ib, eine Verbindung der obigen allgemeinen Formel Ia mit einem Keton der allgemeinen Formel

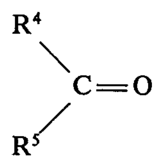

(V)

worin $R^4$ und $R^5$ die in Anspruch 1 angegebene Bedeutung besitzen, umsetzt, oder

d) erwünschtenfalls ein erhaltenes Gemisch von Diastereoisomeren in die diastereoisomeren Racemate auftrennt, und/oder

e) erwünschtenfalls ein erhaltenes Racemat in die optischen Antipoden spaltet, und/oder

f) erwünschtenfalls eine Verbindung der allgemeinen Formel I in ein Salz überführt, oder ein Salz einer Verbindung der allgemeinen Formel I in eine Verbindung der allgemeinen Formel I überführt.

18. Verfahren gemäß Anspruch 17 zur Herstellung von Verbindungen der in Anspruch 1 definierten allgemeinen Formel I, worin entweder R niederes Alkyl und $R^1$ Wasserstoff oder niederes Alkyl bedeuten, oder R und $R^1$ zusammen Trimethylen, Tetramethylen oder Pentamethylen bedeuten, dadurch gekennzeichnet, daß man ein Säureadditionssalz einer Verbindung der Formel II mit einer Säure der Formel III umsetzt, oder eine Verbindung der Formel Ib in Wasser erhitzt, oder einer Verbindung der Formel Ia mit einem Keton der Formel V umsetzt.

19. Arzneimittel, enthaltend eine Verbindung gemäß einem der Ansprüche 1 bis 12.

20. Arzneimittel zur Behandlung von Entzündungen, degenerativen Gelenkkrankheiten oder der Wilson-Krankheit, enthaltend eine Verbindung gemäß einem der Ansprüche 1 bis 12.

**Patentansprüche für den Vertragsstaat AT**

1. Verfahren zur Herstellung von substituierten Phosphin- und Phosphonsäuren der allgemeinen Formel

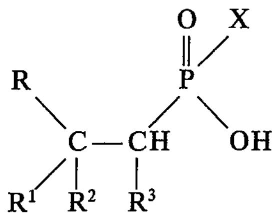

(I)

worin entweder R niederes Alkyl und $R^1$ Wasserstoff oder niederes Alkyl bedeuten, oder R und $R^1$ zusammen mit dem verbindenden Kohlenstoffatom einen 3- bis 6gliedrigen Cycloalkanring bedeuten, und entweder $R^2$ Mercapto und $R^3$ Amino bedeuten, oder $R^2$ und $R^3$ zusammen die Gruppe

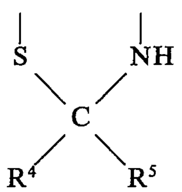

bedeuten, $R^4$ und $R^5$ je niederes Alkyl und X Wasserstoff, Hydroxy, niederes Alkyl oder Aryl bedeuten, und von deren Salzen, dadurch gekennzeichnet, daß man

a) zur Herstellung eines Säureadditionssalzes einer Verbindung der allgemeinen Formel

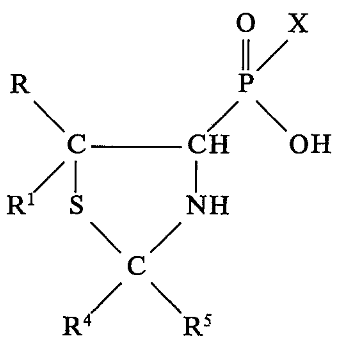

(Ib)

worin R, $R^1$, $R^4$, $R^5$ und X obige Bedeutung besitzen, ein Säureadditionssalz einer Verbindung der allgemeinen Formel

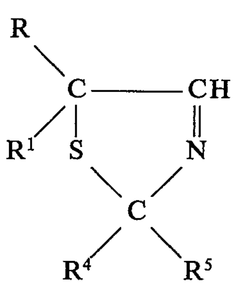

(II)

worin R, $R^1$, $R^4$ und $R^5$ obige Bedeutung besitzen, mit einer Säure der allgemeinen Formel

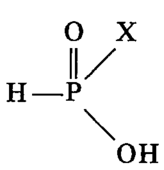

(III)

worin X obige Bedeutung besitzt, umsetzt, oder

b) zur Herstellung einer Verbindung der allgemeinen Formel

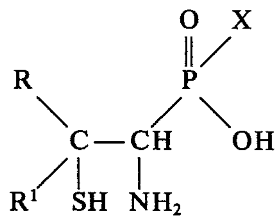

(Ia)

worin R, $R^1$ und X die in Anspruch 1 angegebene Bedeutung besitzen, oder von Säureadditionssalzen davon, in einer Verbindung der Formel Ib oder in einer Verbindung der allgemeinen Formel

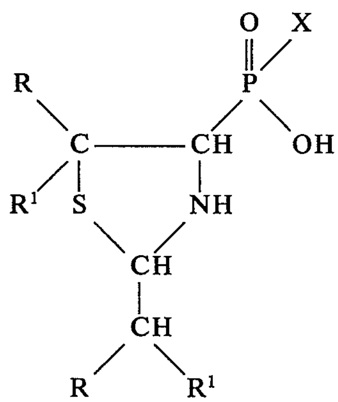

(IV)

worin R, $R^1$ und X obige Bedeutung besitzen, oder in einem Säureadditionssalz einer Verbindung der Formel Ib oder IV den Thiazolidinring spaltet, oder

c) zur Herstellung einer Verbindung der obigen allgemeinen Formel Ib, eine Verbindung der obigen allgemeinen Formel Ia mit einem Keton der allgemeinen Formel

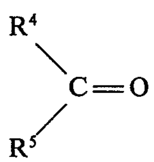

(V)

worin $R^4$ und $R^5$ obige Bedeutung besitzen, umsetzt, oder

d) erwünschtenfalls ein erhaltenes Gemisch von Diastereoisomeren in die diastereoisomeren Racemate auftrennt, und/oder

e) erwünschtenfalls ein erhaltenes Racemat in die optischen Antipoden spaltet, und/oder

f) erwünschtenfalls eine Verbindung der allgemeinen Formel I in ein Salz überführt, oder ein Salz einer Verbindung der allgemeinen Formel I in eine Verbindung der allgemeinen Formel I überführt.

2. Verfahren gemäß Anspruch 1, dadurch gekennzeichnet, daß man Verbindungen der allgemeinen Formel

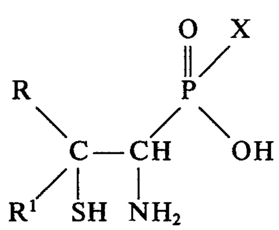

(Ia)

worin R, $R^1$ und X die in Anspruch 1 angegebene Bedeutung besitzen, herstellt.

3. Verfahren gemäß Anspruch 1 oder 2, dadurch gekennzeichnet, daß man Verbindungen der allgemeinen Formel

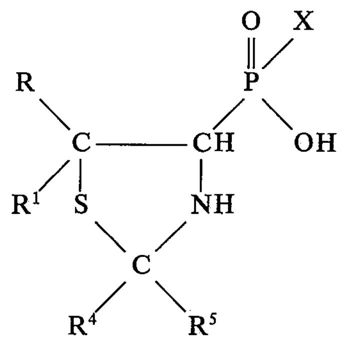

(Ib)

worin R, $R^1$, $R^4$, $R^5$ und X die in Anspruch 1 angegebene Bedeutung besitzen, herstellt.

4. Verfahren gemäß einem der Ansprüche 1 bis 3 zur Herstellung von Verbindungen der in Anspruch 1 definierten allgemeinen Formel I, worin entweder R niederes Alkyl und $R^1$ Wasserstoff oder niederes Alkyl bedeuten, oder R und $R^1$ zusammen Trimethylen, Tetramethylen oder Pentamethylen bedeuten, dadurch gekennzeichnet, daß man ein Säureadditionssalz einer Verbindung der Formel II mit einer Säure der Formel III umsetzt, oder eine Verbindung der Formel Ib in Wasser erhitzt, oder eine Verbindung der Formel Ia mit einem Keton der Formel V umsetzt.

5. Verfahren gemäß einem der Ansprüche 1 bis 4, dadurch gekennzeichnet, daß man Verbindungen der in Anspruch 1 definierten allgemeinen Formel I, worin R und $R^1$ je niederes Alkyl bedeuten, herstellt.

6. Verfahren gemäß Anspruch 2, dadurch gekennzeichnet, daß man DL-(1-Amino-2-mercapto-2-methylpropyl)phosphinsäure herstellt.

7. Verfahren gemäß Anspruch 2, dadurch gekennzeichnet, daß man DL-(1-Amino-2-mercapto-2-methylpropyl)phosphonsäure herstellt.

8. Verfahren gemäß Anspruch 2, dadurch gekennzeichnet, daß man DL-(1-Amino-2-mercapto-2-methylpropyl)methylphosphinsäure herstellt.

9. Verfahren gemäß Anspruch 3, dadurch gekennzeichnet, daß man DL-(2,2,5,5-Tetramethyl-4-thiazolidinyl)phosphinsäure herstellt.

10. Verfahren gemäß Anspruch 3, dadurch gekennzeichnet, daß man DL-(2,2,5,5-Tetramethyl-4-thiazolidinyl)phosphonsäure herstellt.

11. Verfahren gemäß Anspruch 3, dadurch gekennzeichnet, daß man DL-(2,2,5,5-Tetramethyl-4-thiazolidinyl)methylphosphinsäure herstellt.

**Claims for the contracting states: BE, CH, LI, DE, FR, GB, IT, LU, NL, SE**

1. Substituted phosphinic and phosphonic acids of the general formula

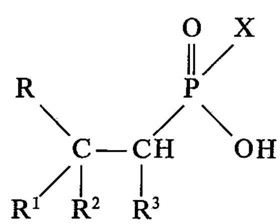

(I)

wherein either R signifies lower alkyl and $R^1$ signifies hydrogen or lower alkyl, or R and $R^1$ together with the linking carbon atom signify a 3- to 6-membered cycloalkane ring, and either $R^2$ signifies mercapto and $R^3$ signifies amino, or $R^2$ and $R^3$ together signify the group

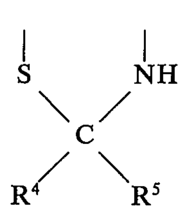

$R^4$ and $R^5$ each signify lower alkyl and X signifies hydrogen, hydroxy, lower alkyl or aryl, and salts thereof in the form of diasteroisomeric mixtures, racemates and optically uniform substances.

2. Compounds of the general formula

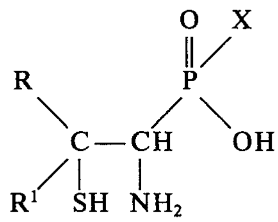

(Ia)

wherein R, $R^1$ and X have the significance given in claim 1.

3. Compounds of the general formula

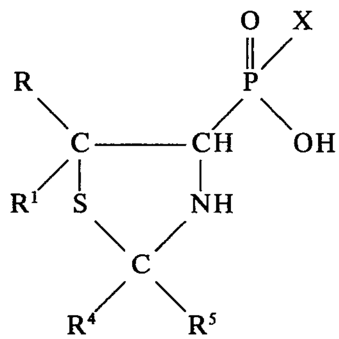

(Ib)

wherein R, $R^1$, $R^4$, $R^5$ and X have the significance given in claim 1.

4. Compounds in accordance with any one of claims 1 to 3, characterized in that either R signifies lower alkyl and $R^1$ signifies hydrogen or lower alkyl, or R and $R^1$ together signify trimethylene, tetramethylene or pentamethylene.

5. Compounds in accordance with any one of claims 1 to 4, chacterized in that R and $R^1$ each signify lower alkyl.

6. DL-(1-Amino-2-mercapto-2-methylpropyl)phosphinic acid.

7. DL-(1-Amino-2-mercapto-2-methylpropyl)phosphinic acid.

8. DL-(1-Amino-2-mercapto-2-methylpropyl)methyl-phosphonic acid.

9. DL-(2,2,5,5-Tetramethyl-4-thiazolidinyl)phosphinic acid.

10. DL-(2,2,5,5-Tetramethyl-4-thiazolidinyl)phosphonic acid.

11. DL-(2,2,5,5-Tetramethyl-4-thiazolidinyl)methylphosphinic acid.

12. DL-(1-Amino-2-mercapto-2-methylpropyl)phenyl-phosphinic acid, DL-(1-amino-2-mercapto-2-methylpropyl)-butylphosphonic acid, (−)-(1-amino-2-mercapto-2-methylpropyl)phosphonic acid, (+)-(1-amino-2-mercapto-2-methylpropyl)phosphinic acid, DL-(2,2,5,5-tetramethyl-4-thiazolidinyl)phenylphosphinic acid and DL-(2,2,5,5-tetramethyl-4-thiazolidinyl)butylphosphinic acid.

13. Compounds of the general formula

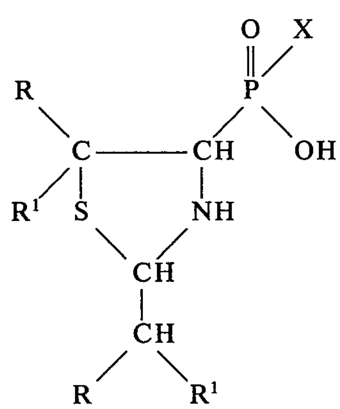

(IV)

wherein either R signifies lower alkyl and $R^1$ signifies hydrogen or lower alkyl, or R and $R^1$ together with the linking carbon atom signify a 3- to 6-membered cycloalkane ring, and X signifies hydrogen, hydroxy, lower alkyl or aryl,

and acid addition salts thereof.

14. Racemic (2-isopropyl-5,5-dimethyl-4-thiazolidinyl)-phosphinic acid.

15. Compounds in accordance with any one of claims 1 to 12 for use as pharmaceutically active substances.

16. Compounds in accordance with any one of claims 1 to 12 as pharmaceutically active substance for the treatment of inflammations, degenerative joint diseases or Wilson's disease.

17. Process for the manufacture of compounds in accordance with any one of claims 1 to 12, characterized by

a) for the manufacture of an acid addition salt of a compound of the general formula

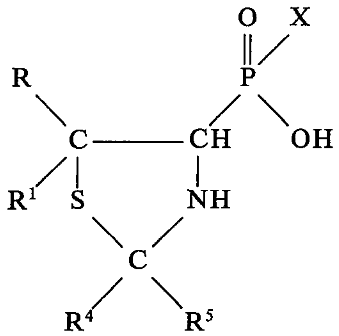

(Ib)

wherein R, $R^1$, $R^4$, $R^5$ and X have the significance given in claim 1, reacting an acid addition salt of a compound of the general formula

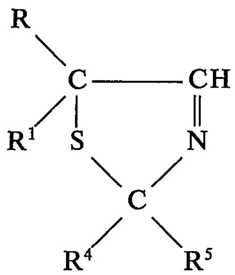

(II)

wherein R, $R^1$, $R^4$ and $R^5$ have the significance given in claim 1, with an acid of the general formula

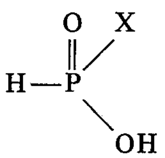

(III)

wherein X has the significance in claim 1,
or

b) for the manufacture of a compound of the general formula

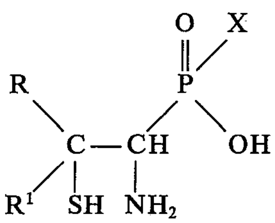

(Ia)

wherein R, $R^1$ and X have the significance given in claim 1, of of acid addition salts thereof, cleaving the thiazolidine ring in a compound of formula Ib or in a compound of the general formula

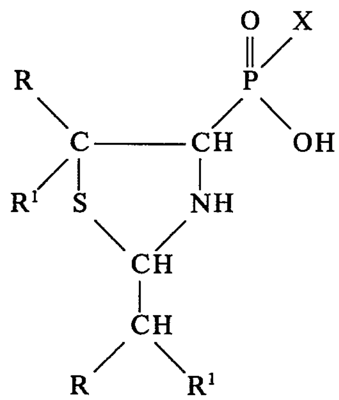

(IV)

wherein R, $R^1$ and X have the significance given in claim 1, or in an acid addition salt of a compound of formula Ib or IV, or

c) for the manufacture of a compound of general formula Ib above, reacting a compound of general formula Ia above with a ketone of the general formula

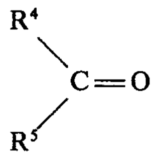

(V)

wherein $R^4$ and $R^5$ have the significance given in claim 1, or

d) if desired, separating a mixture of diastereoisomers obtained into the diastereoisomeric racemates, and/or

e) if desired, resolving a racemate obtained into the optical antipodes, and/or

f) if desired, converting a compound of general formula I into a salt, or converting a salt of a compound of general formula I into a compound of general formula I.

18. Process in accordance with claim 17 for the manufacture of compounds of general formula I defined in claim 1 wherein either R signifies lower alkyl and $R^1$ signifies hydrogen or lower alkyl, or R and $R^1$ together signify trimethylene, tetramethylene or pentamethylene, characterized by reacting an acid addition salt of a compound of formula II with an acid of formula III, or heating a compound of formula Ib in water, or reacting a compound of formula Ia with a ketone of formula V.

19. Medicament, containing a compound in accordance with any one of claims 1 to 12.

20. Medicament for the treatment of inflammations, degenerative joint diseases or Wilson's disease, containing a compound in accordance with any one of claims 1 to 12.

**Claims for the Contracting state: AT**

1. Process for the manufacture of substituted phosphinic and phosphonic acids of the general formula

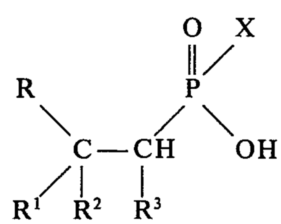

(I)

wherein either R signifies lower alkyl and $R^1$ signifies hydrogen or lower alkyl, or R and $R^1$ together with the linking carbon atom signify a 3- to 6-membered cycloalkane ring, and either $R^2$ signifies mercapto and $R^3$ signifies amino, or $R^2$ and $R^3$ together signify the group

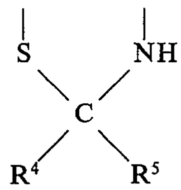

$R^4$ and $R^5$ each signify lower alkyl and X signifies hydrogen, hydroxy, lower alkyl or aryl, and of their salts, characterized by

a) for the manufacture of an acid addition salt of a compound of the general formula

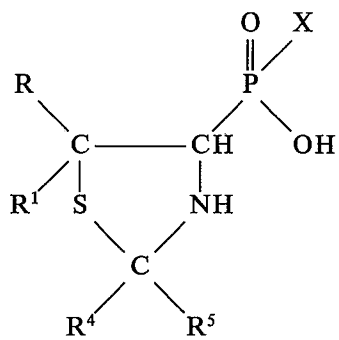

(Ib)

wherein R, $R^1$, $R^4$, $R^5$ and X have the above significance,
reacting an acid addition salt of a compound of the general formula

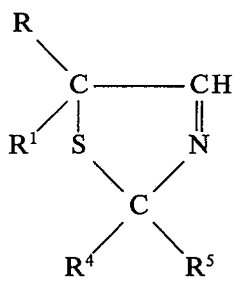

(II)

wherein R, $R^1$, $R^4$ and $R^5$ have the above significance,
with an acid of the general formula

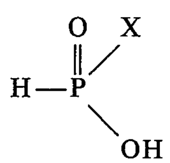

(III)

wherein X has the above significance,
or

b) for the manufacture of a compound of the general formula

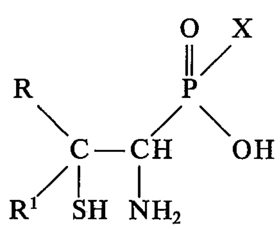

(Ia)

wherein R, $R^1$ and X have the above significance,
or of acid addition salts thereof, cleaving the thiazolidine ring in a compound of formula Ib or in a compound of the general formula

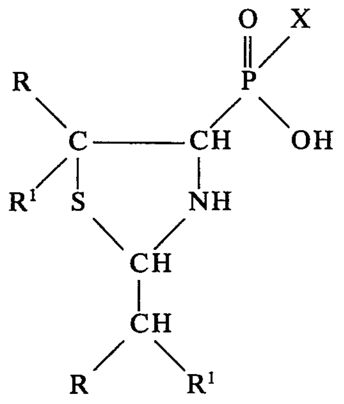

(IV)

wherein R, $R^1$ and X have the above significance
or in an acid addition salt of a compound of formula Ib or IV, or

c) for the manufacture of a compound of general formula Ib above, reacting a compound of general formula Ia above with a ketone of the general formula

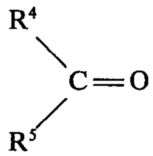

(V)

wherein $R^4$ and $R^5$ have the above significance,
or

d) if desired, separating a mixture of diastereoisomers obtained into the diastereoisomeric racemates, and/or
e) if desired, resolving a racemate obtained into the optical antipodes, and/or
f) if desired, converting a compounds of general formula I into a salt, or converting a salt of a compound of general formula I into a compound of general formula I.

2. Process in accordance with Claim 1, characterized by preparing compounds of the general formula

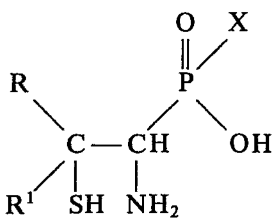

(Ia)

wherein R, $R^1$ and X have the significance given in claim 1.

3. Process in accordance with Claim 1 or 2, characterized by preparing compounds of the general formula

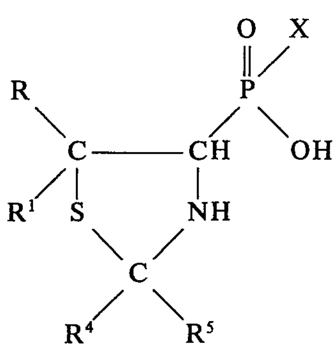

(Ib)

wherein R, $R^1$, $R^4$, $R^5$ and X habe the significance given in claim 1.

4. Process in accordance with any one of Claims 1 to 3 for the manufacture of compounds of general formula I defined in claim 1 wherein either R signifies lower alkyl and $R^1$ signifies hydrogen or lower alkyl, or R and $R^1$ together signify trimethylene, tetramethylene or pentamethylene, characterized by reacting an acid addition salt of a compound of formula II with an acid of formula III, or heating a compound of formula Ib in water, or reacting a compound of formula Ia with a ketone of formula V.

5. Compounds in accordance with any one of Claims 1 to 4, characterized by preparing compounds of general formula I defined in Claim 1 wherein R and $R^1$ each signify lower alkyl.

6. Process in accordance with Claim 2, characterized by preparing DL-(1-Amino-2-mercapto-2-methylpropyl)phosphinic acid.

7. Process in accordance with Claim 2, characterized by preparing DL-(1-Amino-2-mercapto-2-methylpropyl)phosphonic acid.

8. Process in accordance with Claim 2, characterized by preparing DL-(1-Amino-2-mercapto-2-methylpropyl)methyl-phosphinic acid.

9. Process in accordance with Claim 3, characterized by preparing DL-(2,2,5,5-Tetramethyl-4-thiazolidinyl)phosphinic acid.

10. Process in accordance with Claim 3, characterized by preparing DL-(2,2,5,5-Tetramethyl-4-thiazolidinyl)phosphonic acid.

11. Process in accordance with Claim 3, characterized by preparing DL-(2,2,5,5-Tetramethyl-4-thiazolidinyl)methyl-phosphinic acid.

**Revendications pour les Etats contractants: BE, CH, LI, DE, FR, GB, IT, LU, NL, SE**

1. Acides phosphiniques et phosphoniques substitués de formule générale

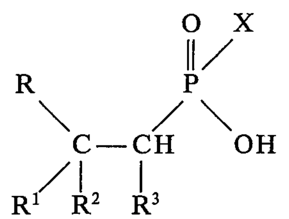

(I)

où ou bien R représente un alcoyle inférieur et $R^1$ un hydrogène ou un alcoyle inférieur, ou bien R et $R^1$ représentent avec l'atome de carbone qui les relie un noyau cycloalcane à 3 à 6 chaînons, et ou bien $R^2$ représente un mercapto et $R^3$ un amino, ou bien $R^2$ et $R^3$ représentent ensemble le groupe

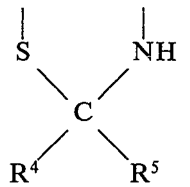

$R^4$ et $R^5$ représentent chacun un alcoyle inférieur, et X représente un hydrogène, un hydroxy, un alcoyle inférieur ou un aryle, et leurs sels sous forme de mélanges diastéréoisomériques, de racémates et de corps optiquement uniformes.

2. Composés de formule générale

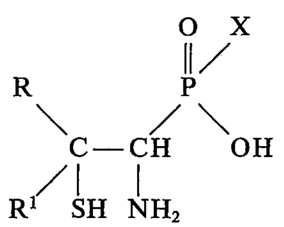

(Ia)

où R, $R^1$ et X ont la signification donnée dans la revendication 1.

3. Composés de formule générale

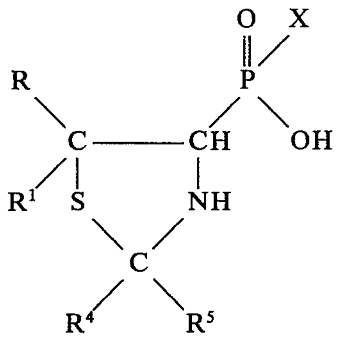

(Ib)

où R, $R^1$, $R^4$, $R^5$ et X ont la signification donnée dans la revendication 1.

4. Composés selon l'une des revendications 1 à 3, caractérisés en ce que ou bien R représente un alcoyle inférieur et $R^1$ un hydrogène ou un alcoyle inférieur, ou bien R et $R^1$ représentent ensemble un triméthylène, un tétraméthylène ou un pentaméthylène.

5. Composés selon l'une des revendications 1 à 4, caractérisés en ce que R et $R^1$ représentent chacun un alcoyle inférieur.

6. Acide DL-(1-amino-2-mercapto-2-méthylpropyl)phosphinique.

7. Acide DL-(1-amino-2-mercapto-2-méthylpropyl)-phosphonique.

8. Acide DL-(1-amino-2-mercapto-2-méthylpropyl)méthylphosphinique.

9. Acide DL-(2,2,5,5-tétraméthyl-4-thiazolidinyl)phosphinique.

10. Acide DL-(2,2,5,5-tétraméthyl-4-thiazolidinyl)phosphonique.

11. Acide DL-(2,2,5,5-tétraméthyl-4-thiazolidinyl)méthylphosphinique.

12. Acide DL-(1-amino-2-mercapto-2-méthylpropyl)phénylphosphinique, acide DL-(1-amino-2-mercapto-2-méthylpropyl)butyl-phosphinique, acide (−)-(1-amino-2-mercapto-2-méthylpropyl)-phosphinique, acide (+)-(1-amino-2-mercapto-2-méthylpropyl)-phosphinique, acide DL-(2,2,5,5-tétraméthyl-4-thiazolidinyl)-phénylphosphinique et acide DL-(2,2,5,5-tétraméthyl-4-thiazolidinyl)butylphosphinique.

13. Composés de formule générale

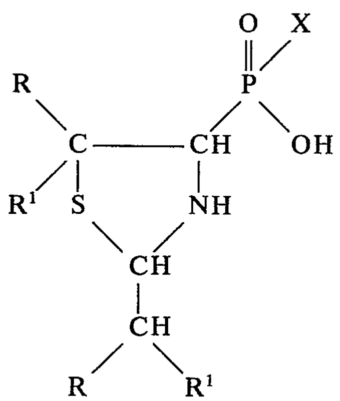

(IV)

où ou bien R représente un alcoyle inférieur et $R^1$ un hydrogène ou un alcoyle inférieur, ou bien R et $R^1$ représentent ensemble avec l'atome de carbone qui les relie un noyau cycloalcane à 3 à 6 chaînons, et X représente un hydrogène, un hydroxy, un alcoyle inférieur ou un aryle, et leurs sels d'addition d'acides.

14. Acide (2-isopropyl-5,5-diméthyl-4-thiazolidinyl)phosphinique racémique.

15. Composés selon l'une des revendications 1 à 12 aux fins d'application comme substances actives pharmaceutiques.

16. Composés selon l'une des revendications 1 à 12 comme substance actives pharmaceutiques pour le traitement des inflammations, des maladies dégénératives des articulations ou de la maladie de Wilson.

17. Procédé de préparation de composés selon l'une des revendications 1 à 12, caractérisé en ce que

a) pour préparer un sel d'addition d'acide d'un composé de formule générale

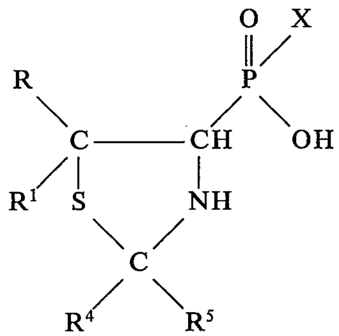

(Ib)

où R, $R^1$, $R^4$, $R^5$ et X ont la signification donnée dans la revendication 1, on fait réagir un sel d'addition d'acide d'un composé de formule générale

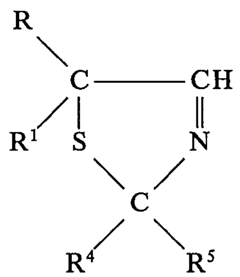

(II)

où R, $R^1$, $R^4$ et $R^5$ ont la signification donnée dans la revendication 1, avec un acide de formule générale

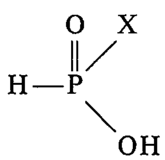

(III)

où X a la signification donnée dans la revendication 1, ou

b) pour préparer un composé de formule générale

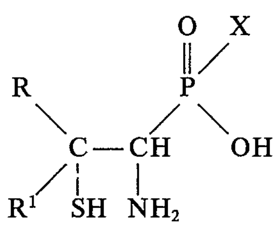

(Ia)

où R, $R^1$ et X ont la signification donnée dans la revendication 1, ou un de ses sels d'addition d'acides, dans un composé de formule Ib ou dans un composé de formule générale

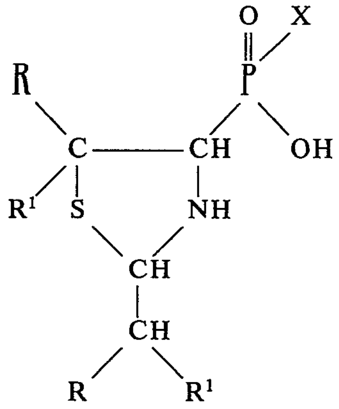

(IV)

où R, $R^1$ et X ont la signification donnée dans la revendication 1,
ou dans un sel d'addition d'acide d'un composé de formule Ib ou IV, on sépare le noyau thiazolidino, ou

c) pour préparer un composé de formule générale Ib ci-dessus,
on fait réagir un composé de formule générale Ia ci-dessus avec une cétone de formule générale

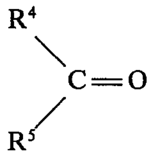

(V)

où $R^4$ et $R^5$ ont la signification donnée dans la revendication 1, ou

d) si on le désire on dédouble un mélange de diastéréoisomères obtenu pour donner les racémates diastéréoisomère, et/ou
e) si on le désire on dédouble un racémate obtenu pour donner les antipodes optiques, et/ou
f) si on le désire on transforme un composé de formule générale I en un sel, ou on transforme un sel d'un composé de formule générale I en un composé de formule générale I.

18. Procédé selon la revendication 17 de préparation de composés de formule générale I définie dans la revendication 1, ou où bien R représente un alcoyle inférieur et $R^1$ un hydrogène ou un alcoyle inférieur, ou bien R et $R^1$ représentent ensemble un triméthylène, un tétraméthylène ou un pentaméthylène, caractérisé en ce qu'on fait réagir un sel d'addition d'acide d'un composé de formule II avec un acide de formule III, ou en ce qu'on chauffe un composé de formule Ib dans l'eau, ou en ce qu'on fait réagir un composé de formule Ia avec une cétone de formule V.

19. Médicament contenant un composé selon l'une des revendications 1 à 12.

20. Médicament pour le traitement des inflammations, des maladies dégénératives des articulations ou de la maladie de Wilson, contenant un composé selon l'une des revendications 1 à 12.

**Revendications pour l'Etat contractant: AT**

1. Procédé de préparation d'acides phosphiniques et phosphoniques substitués de formule générale

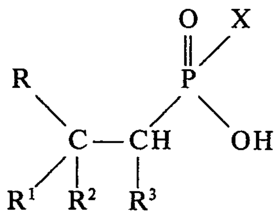

(I)

où ou bien R représente un alcoyle inférieur et $R^1$ un hydrogène ou un alcoyle inférieur, ou bien R et $R^1$ représentent avec l'atome de carbone qui les relie un noyau cycloalcane à 3 à 6 chaînons, et ou bien $R^2$ représente un mercapto et $R^3$ un amino, ou bien $R^2$ et $R^3$ représentent ensemble le groupe

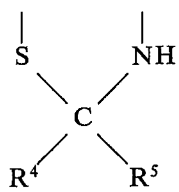

$R^4$ et $R^5$ représentent chacun un alcoyle inférieur, et X un hydrogène, un hydroxy, un alcoyle inférieur ou un aryle,
et de leurs sels, caractérisé en ce que

a) pour préparer un sel d'addition d'acide d'un composé de formule générale

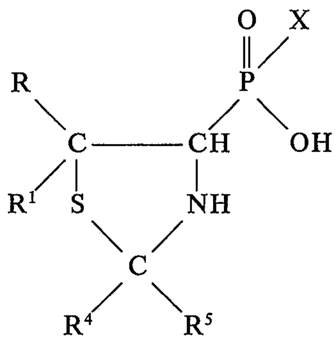

(Ib)

où R, $R^1$, $R^4$, $R^5$ et X ont la signification donnée ci-dessus,
on fait réagir un sel d'addition d'acide d'un composé de formule générale

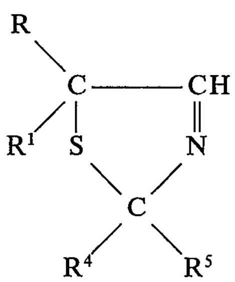

(II)

où R, $R^1$, $R^4$ et $R^5$ ont la signification donnée ci-dessus,
avec un acide de formule générale

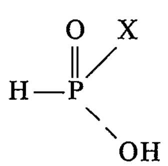

(III)

où X a la signification donnée ci-dessus, ou

b) pour préparer un composé de formule générale

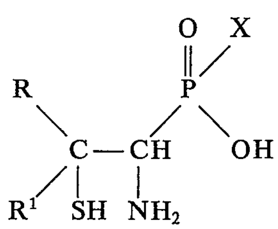

(Ia)

où R, $R^1$ et X ont la signification donnée ci-dessus,
ou ses sels d'addition d'acides, dans un composé de formule Ib ou dans un composé de formule générale

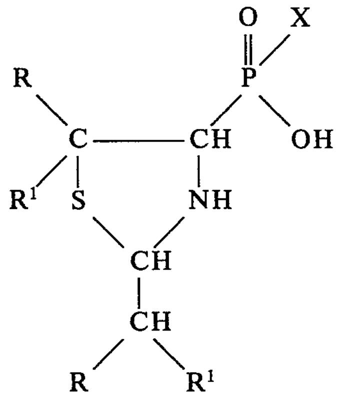

(IV)

où R, $R^1$ et X ont la signification donnée ci-dessus, ou dans un sel d'addition d'acide d'un composé de formule Ib ou IV, on sépare le noyau thiazolidine, ou

c) pour préparer un composé de formule générale Ib ci-dessus, on fait réagir un composé de formule générale Ia ci-dessus avec une cétone de formule générale

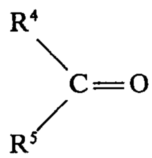

(V)

où $R^4$ et $R^5$ ont la signification donnée ci-dessus, ou

d) si on le désire on dédouble un mélange de diastéréoisomères obtenu pour donner les racémates diastéréoisomères, et/ou

e) si on le désire on sépare un racémate obtenu pour donner les antipodes optiques, et/ou

f) si on le désire on transforme un composé de formule générale I en un sel, ou on transforme un sel d'un composé de formule générale I en un composé de formule générale I.

2. Procédé selon la revendication 1, caractérisé en ce qu'on prépare des composés de formule générale

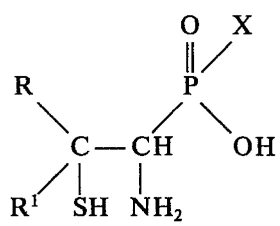

(Ia)

où R, $R^1$ et X ont la signification donnée dans la revendication 1.

3. Prodédé selon l'une des revendications 1 ou 2, caractérisé en ce qu'on prépare des composés de formule générale

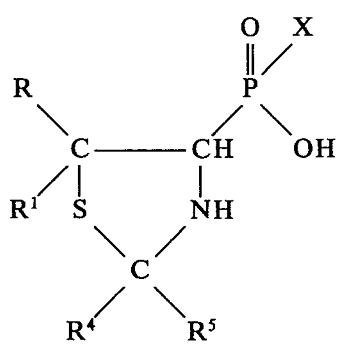

(Ib)

où R, $R^1$, $R^4$, $R^5$ et X ont la signification donnée dans la revendication 1.

4. Procédé selon l'une des revendication 1 à 3 de préparation de composés de formule générale I

définie dans la revendication 1, où bien R représente un alcoyle inférieur et $R^1$ un hydrogéne ou un alcoyle inférieur, ou bien R et $R^1$ représentent ensemble un triméthylène, un tétraméthylène ou un pentaméthylène, caractérisé en ce qu'on fait réagir un sel d'addition d'acide d'un composé de formule II avec un acide de formule III, ou en ce qu'on chauffe composé de formule Ib dans l'eau, ou en ce qu'on fait réagir un composé de formule Ia avec une cétone de formule V.

5. Procédé selon l'une des revendications 1 à 4, caractérisé en ce qu'on prépare des composés de formule générale I définie dans la revendication 1, où R et $R^1$ représentent chacun un alcoyle inférieur.

6. Procédé selon la revendication 2, caractérisé en ce qu'on prépare l'acide DL-(1-amino-2-mercapto-2-méthylpropyl)-phosphinique.

7. Procédé selon la revendication 2, caractérisé en ce qu'on prépare l'acide DL-(1-amino-2-mercapto-2-méthylpropyl)-phosphonique.

8. Procédé selon la revendication 2, caractérisé en ce qu'on prépare l'acide DL-(1-amino-2-mercapto-2-méthylpropyl)-méthyl-phosphinique.

9. Procédé selon la revendication 3, caractérisé en ce qu'on prépare l'acide DL-(2,2,5,5-tétraméthyl-4-thiazolidinyl)-phosphinique.

10. Procédé selon la revendication 3, caractérisé en ce qu'on prépare l'acide DL-(2,2,5,5-tétraméthyl-4-thiazolidinyl)-phosphinique.

11. Procédé selon la revendication 3, caractérisé en ce qu'on prépare l'acide DL-(2,2,5,5-tétraméthyl-4-thiazolidinyl)-méthyl-phosphinique.